# EUROPÄISCHE PATENTANMELDUNG

(11) **EP 4 417 243 A1**
(43) Veröffentlichungstag der Anmeldung: **21.08.2024**
(21) Anmeldenummer: 23156684.5
(22) Anmeldetag: 15.02.2023
(51) Int. Cl.: A61M 39/02, A61M 39/04, A61M 31/00

(54) **IMPLANTAT ZUR VERZÖGERTEN WIRKSTOFFFREISETZUNG**

(71) Anmelder: Heraeus Medical GmbH, 61273 Wehrheim (DE)
(72) Erfinder: DR. VOGT, Sebastian, 61273 Wehrheim (DE); DR. KLUGE, Thomas, 61273 Wehrheim (DE)
(74) Vertreter: Heraeus IP

(57) **Zusammenfassung**

Die Erfindung betrifft ein Implantat zur subkutanen verzögerten Wirkstofffreisetzung, aufweisend ein Gehäuse, welches an einer ersten Seite durch ein Septum begrenzt ist, und an einer zweiten Seite einen Auslass zur Wirkstofffreisetzung aufweist, und einen Speicher, der in einem Hohlraum des Gehäuses angeordnet und zur Aufnahme einer wirkstoffhaltigen Flüssigkeit eingerichtet ist.

## Beschreibung

### GEBIET DER ERFINDUNG

Die vorliegende Erfindung betrifft das Gebiet der Medizintechnik, insbesondere implantierbare Vorrichtungen zur Wirkstoffabgabe mit verzögerter Freisetzung. Die Implantate können subkutan in räumlicher Nähe zu Gelenken, beispielsweise Kniegelenken, implantiert werden. Mögliche Anwendungen liegen beispielsweise in der Behandlung von entzündlichen Gelenkerkrankungen, wie zum Beispiel rheumatoider Arthritis.

### TECHNISCHER HINTERGRUND

Rheumatische Erkrankungen gehören zu den sogenannten Autoimmunerkrankungen, bei denen das Immunsystem aus gegenwärtig noch nicht genau bekannten Ursachen körpereigene Strukturen angreift. Die Erkrankungen rheumatoide Arthritis und die Psoriasisarthritis gehören zum rheumatischen Formenkreis und sind mit Gelenkentzündungen (Arthritiden) verbunden. Diese meist chronisch verlaufenden Gelenkentzündungen können zur fortschreitenden Zerstörung der Gelenke führen. Die Gelenke des Menschen sind von einer Gelenkkapsel (Capsula articulatis) umgeben. Diese setzt sich aus einer äußeren Bindegewebsschicht (Membrana fibrosa) und einer inneren Schicht der sogenannten Synovialmembran (Membrana synovialis) zusammen. Die Synovialmembran ist für die Entzündungsprozesse bei der rheumatoiden Arthritis und der Psoriasisarthritis entscheidend, weil durch diese hindurch fehlgesteuerte Immunzellen einwandern, beziehungsweise weil durch die Proliferation von Fibroblasten auf der Synovialmembran ein Pannus - das ist ein Zellklon von Fibroblasten - gebildet werden kann. Beim Zerfall des Pannus und durch die eingewanderten Immunzellen werden Entzündungsmediatoren wie Interleukine und α-TNF (α-Tumornekrosefaktor) sezerniert. Diese Entzündungsmediatoren steuern über eine Entzündungskaskade die Freisetzung von Proteasen und anderen destruierenden Enzymen aus Immunzellen, welche dann zu einer enzymatischen Zerstörung der Knorpel- und Knochenstrukturen führen.

Es sind bereits Implantate zur Abgabe von pharmazeutischen Wirkstoffen im Bereich der Gelenke bekannt, die zur lokalen Applikation von Antibiotika eingesetzt werden. Beispielhaft seien hierfür die Dokumente GB 2 290 971 A, US 2010/0042213 A, WO 2017/178951 A1, WO 2007/084878 A1 und US 6 245 111 B1 genannt. Beim Einsatz dieser Implantate muss aber das Gelenk großflächig entfernt werden und die Implantate dienen vornehmlich der Behandlung der Prothesen mit Antibiotika. Derartige Implantate zur Behandlung einer rheumatoiden Arthritis oder einer Psoriasisarthritis einzusetzen, kommt nicht in Betracht, weil das zu behandelnde Gelenk für die Therapie entfernt werden müsste und dadurch die nachfolgende Behandlung sinnlos wäre. Die US 2007/0219471 A1 offenbart ein Implantat zum Applizieren von Wirkstoffen und Aufrechterhalten eines reduzierten Drucks zur Beschleunigung der Wundheilung in und zur mechanischen Stabilisierung von schlecht heilenden Kavitäten.

Aus der WO 2010/088548 A1 ist ein röhrenförmiges, wiederauffüllbares Augen-Implantat bekannt, mit dem ein pharmazeutischer Wirkstoff direkt an den Augapfel abgegeben werden kann. Hierzu wird das röhrenförmige Implantat direkt in den Augapfel eingesteckt. Für eine Implantation in ein Gelenk ist das Implantat nicht vorgesehen und auch nicht geeignet. Durch das Einsetzen im Gelenk würde das Gelenk selbst angegriffen und beschädigt und bei Einsetzen des Implantats bestünde das Risiko einer Infektion und Entzündung des Gelenks.

In EP3978065 ist ein scheibenförmiges Implantat aus einem elastischen Material bekannt, das einen Hohlraum besitzt, der mit einer Kanüle mit einer Wirkstofflösung befüllt werden kann, wobei sich das Implantat elastisch ausdehnt. Die Wirkstofflösung tritt infolge der Rückstellkraft des Implantats aus Mikrobohrungen an der Unterseite des Implantats aus.

Die US 5 681 289 A und die US 2018/0028320 A1 offenbaren Implantate, bei denen eine medizinische Flüssigkeit zur Behandlung von Gelenken oder zum Schmieren von Gelenken aus einem Reservoir über Schläuche den Gelenken zugeführt wird. Die Implantate sind relativ groß und nur aufwendig zu platzieren. Eine kurzfristige Änderung einer medizinischen Behandlung ist aufwendig, weil die Zuleitungen zu den flächenförmigen Applikatoren zuerst gespült werden müssten, was im implantierten Zustand nicht ohne weiteres möglich ist. Für das Schmieren von Gelenken mit einem über das Implantat abgegebenen Schmiermittel sind die Implantate gut geeignet, während eine gezielte Abgabe von pharmazeutischen Wirkstoffen mit hoher Konzentration mit Hilfe dieser Implantate nicht möglich erscheint, weil in dem Implantat, dem Reservoir und den Zuleitung zu große Volumina enthalten sind.

In der US 2003/0139811 A1 ist ein Wirkstoffsystem zur lokalen Freisetzung von entzündungshemmenden Wirkstoffen im Innenraum von Gelenkskapseln offenbart. Dabei handelt es sich um hohle Schrauben mit einer Öffnung im Schraubenkopf. Diese hohlen Schrauben können mit einem Wirkstoff befüllt werden. Anschließend werden die Schrauben in Knochengewebe eingebracht, das sich neben dem Knorpelgewebe innerhalb der Gelenkskapsel befindet. Über die Öffnung im Schraubenkopf soll anschließend der Wirkstoff in die Synovialflüssigkeit freigesetzt werden. Kritisch ist hierbei zu sehen, dass die Gelenkskapsel eröffnet werden muss und dass die Schraube nach erfolgter Wirkstofffreisetzung im Innenraum der Gelenkskapsel verbleibt. Eine Eröffnung der Gelenkskapsel ist immer mit einem deutlichen Infektionsrisiko behaftet. Eine Auffüllung der implantierten Schraube mit neuem Wirkstoff ist nicht vorgesehen. Daher kann die Behandlung auch nicht an eine Veränderung des Zustands des behandelten Patienten angepasst werden.

### BEVORZUGTE AUSFÜHRUNGSFORMEN

Die Aufgabe der vorliegenden Erfindung besteht darin, eines oder mehrere der oben geschilderten und weitere Probleme des Stands der Technik zu lösen. Beispielsweise ermöglicht die Erfindung eine verzögerte Freisetzung eines Wirkstoffs aus einem Implantat. Weiterhin liefert die vorliegende Erfindung ein Implantat, das im unveränderten implantierten Zustand mithilfe einer Injektionskanüle mit Wirkstoff befüllt werden kann.

Diese Aufgaben werden gelöst durch die hierin beschriebenen Verfahren und Vorrichtungen, insbesondere denjenigen, die in den Patentansprüchen beschrieben sind.

Bevorzugte Ausführungsformen der Erfindung werden nachstehend beschrieben.

Eine erste Ausführungsform beschreibt ein Implantat zur subkutanen verzögerten Wirkstofffreisetzung, aufweisend ein Gehäuse, welches an einer ersten Seite durch ein Septum begrenzt ist, und an einer zweiten Seite einen Auslass zur Wirkstofffreisetzung aufweist, und einen Speicher, der in einem Hohlraum des Gehäuses angeordnet und zur Aufnahme einer wirkstoffhaltigen Flüssigkeit eingerichtet ist.

Eine zweite Ausführungsform beschreibt ein Implantat nach Ausführungsform 1, wobei das Implantat innerhalb des Gehäuses weiterhin einen Durchstechschutz aufweist, der gemeinsam mit einer Wand des Gehäuses einen Kanal definiert, wobei der Kanal eingerichtet ist, Wirkstoff aus dem Speicher zum Auslass zu leiten.

Eine dritte Ausführungsform beschreibt ein Implantat gemäß der zweiten Ausführungsform, wobei der Durchstechschutz eine Oberflächenstruktur umfasst, welche einen Abstand des Durchstechschutz zu dem Speicher und/oder zu dem Gehäuse definiert. Ein solcher Abstand kann beispielsweise jeweils mindestens 0,5 mm oder mindestens 1 mm betragen.

Eine vierte Ausführungsform beschreibt ein Implantat gemäß einer der vorangehenden Ausführungsformen, wobei der Speicher ein offenporiges Material aufweist.

Eine fünfte Ausführungsform beschreibt ein Implantat gemäß einer der vorangehenden Ausführungsformen, wobei der Speicher einen retikulierten Schaumstoff, gesinterte Kunststoffpartikel, gepresste Kunststofffasern, gepresste Naturstoffasern, oder verklebte Kunststofffasern aufweist.

Eine sechste Ausführungsform beschreibt ein Implantat gemäß einer der vorangehenden Ausführungsformen, wobei der Speicher ein vernetztes Salz der Polyacrylsäure, ein vernetztes Polyacrylamid, ein Salz einer vernetzten Polystyrolsulfonsäure, ein Polysaccharid oder ein Polysaccharidderivat aufweist.

Eine siebte Ausführungsform beschreibt ein Implantat gemäß einer der vorangehenden Ausführungsformen, wobei der Speicher innerhalb des Gehäuses angeordnet und eingerichtet ist, die freie Zirkulation einer wirkstoffhaltigen Flüssigkeit zwischen dem Septum und dem Auslass zu ermöglichen.

Eine achte Ausführungsform beschreibt ein Implantat gemäß einer der vorangehenden Ausführungsformen, wobei das Septum ein flexibles, selbstdichtendes Polymer aufweist, wobei das Polymer bevorzugt ein polyhalogeniertes Olefin, ein Silikon, oder ein thermoplastisches Elastomer aufweist.

Eine neunte Ausführungsform beschreibt ein Implantat gemäß einer der vorangehenden Ausführungsformen, wobei das Septum eine konvex gewölbte Form aufweist.

Eine zehnte Ausführungsform beschreibt dann Implantat gemäß einer der vorangehenden Ausführungsformen, wobei das Septum eingerichtet ist, sich bei Injektion einer Flüssigkeit in den Hohlraum des Gehäuses elastisch zu dehnen.

Eine elfte Ausführungsform beschreibt ein Implantat gemäß einer der vorangehenden Ausführungsformen, wobei die zweite Seite des Gehäuses eine im wesentlichen flache Form aufweist.

Eine zwölfte Ausführungsform beschreibt Implantat gemäß einer der vorangehenden Ausführungsformen, wobei der Innendurchmesser des Auslasses die Abgabegeschwindigkeit eines Wirkstoffs aus dem Speicher bestimmt.

Eine dreizehnte Ausführungsform beschreibt ein Implantat gemäß einer der vorangehenden Ausführungsformen, welches weiterhin ein Befestigungselement aufweist, das zur Verbindung mit Körpergewebe eingerichtet ist.

Eine vierzehnte Ausführungsform beschreibt ein Implantat gemäß einer der vorangehenden Ausführungsformen, wobei der Speicher mit einer wirkstoffhaltigen Flüssigkeit befüllt ist.

Eine fünfzehnte Ausführungsform beschreibt ein Implantat gemäß einer der vorangehenden Ausführungsformen, welches eingerichtet ist, in einem subkutan implantierten Zustand mit einer wirkstoffhaltigen Flüssigkeit befüllt zu werden, indem die Flüssigkeit durch das Septum in das Gehäuse, bevorzugt in den Speicher, injiziert wird.

### AUSFÜHRLICHE BESCHREIBUNG

Zu den hierin beschriebenen Ausführungsformen, deren Elemente ein bestimmtes Merkmal (z.B. ein Material) "aufweisen", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "aufweisen" oder "aufweisend" verwendet.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist.

Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einem Verfahren beschrieben werden, auch für die hierin beschriebenen Erzeugnisse und Vorrichtungen anwendbar sind, und umgekehrt. Lediglich aus Gründen der knapperen Darstellung werden alle diese erwogenen Kombinationen nicht in allen Fällen explizit aufgeführt. Auch technische Lösungen, die zu den hierin beschriebenen Merkmalen bekanntermaßen gleichwertig sind, sollen grundsätzlich vom Umfang der Erfindung umfasst sein.

Ein erster Aspekt der Erfindung betrifft in einer ersten Ausführungsform ein Implantat zur subkutanen verzögerten Wirkstofffreisetzung, aufweisend ein Gehäuse, welches an einer ersten Seite durch ein Septum begrenzt ist, und an einer zweiten Seite einen Auslass zur Wirkstofffreisetzung aufweist, und einen Speicher, der in einem Hohlraum des Gehäuses angeordnet und zur Aufnahme einer wirkstoffhaltigen Flüssigkeit eingerichtet ist.

Das Implantat weist ein Gehäuse auf, welches bevorzugt aus einem biokompatiblen Material gefertigt ist. Das Gehäuse kann ein Kunststoff oder ein Metall umfassen, oder beides. Das Gehäuse kann mehrteilig ausgebildet sein. Das Gehäuse weist ein Septum auf, welches bevorzugt aus einem weichen, dehnbaren Material gebildet ist. Das Septum ist bevorzugt eingerichtet, mithilfe einer medizinischen Kanüle durchstochen zu werden, um eine Flüssigkeit in das Gehäuse zu injizieren. Das Septum ist an einer ersten Seite des Gehäuses angebracht. Diese erste Seite ist bevorzugt dazu eingerichtet, im implantierten Zustand des Implantats, zum Beispiel eine subkutane Implantation, in Richtung der Hautoberfläche ausgerichtet zu werden, sodass das Septum von außen mithilfe einer Kanüle zugänglich ist. Das Gehäuse umfasst einen Hohlraum, in welchem ein Speicher angeordnet ist. Der Speicher ist zur Aufnahme einer wirkstoffhaltigen Flüssigkeit eingerichtet. Dies bedeutet, dass eine wässrige oder ölige Flüssigkeit ins Innere des Speichers gelangen kann. Der Speicher kann hierzu eine offene Porosität aufweisen, wie nachfolgend ausführlicher beschrieben.

Weiterhin weist das Implantat einen Auslass an einer zweiten Seite des Gehäuses auf, um Wirkstoff aus dem Gehäuse nach außen abzugeben. Dies kann durch Diffusion eines in einer wirkstoffhaltigen Flüssigkeit gelösten oder suspendierten Substanz durch den Auslass geschehen, oder durch Fließen einer solcher Flüssigkeit durch den Auslass aus dem Gehäuse, oder beides.

Der Auslass kann ein Ventil oder einen Filter umfassen, um das Eindringen hochmolekularer Stoffe, Keime oder sonstiger Partikel in das Implantat zu verhindern. Beispielsweise kann am Auslass ein Filter mit einem Ausschlussvolumen von 220 nm, 200 nm, 100 nm, 75 nm oder 50 nm angebracht sein. Der Filter kann beispielsweise Polyethersulfon (PES), Polyvinylidenfluorid (PVDF) oder Poltetrafluoethylen (PTFE) aufweisen. Das Ventil oder der Filter können auch die Freisetzungskinetik des Wirkstoffs definieren.

Die zweite Seite des Gehäuses ist gegenüberliegend der ersten Seite des Gehäuses angeordnet. In einem implantierten Zustand des Implantats kann die zweite Seite zum Körperinneren des Patienten ausgerichtet sein, beispielsweise in Richtung eines zu behandelnden Gewebes, zum Beispiel eines Gelenks. Dies erlaubt eine gezielte lokale Wirkstoffabgabe an ein Zielgewebe.

Das Gehäuse kann eine scheibenförmige Form aufweisen. Das Gehäuse kann beispielsweise die Form eines flachen Zylinders aufweisen. Das Gehäuse kann mehrteilig ausgebildet sein, wobei die verschiedenen Teile beispielsweise mithilfe eines Klick- oder Schnappverschluss bzw. Verrastung dauerhaft flüssigkeitsdicht miteinander verbindbar sein können. Das Gehäuse kann, bis auf die Auslässe, flüssigkeitsundurchlässig sein. Das Gehäuse kann ein Metall oder einen Kunststoff aufweisen. Das Gehäuse weist bevorzugt ein biokompatibles Material auf.

In einigen Ausführungsformen weist das Implantat innerhalb des Gehäuses weiterhin einen Durchstechschutz auf. Der Durchstechschutz kann eingerichtet sein, das vollständige Durchstechen des Gehäuses mit einer Kanüle auf der ersten und der zweiten Seite zu verhindern. Der Durchstechschutz kann insbesondere eingerichtet sein, ein Durchstechen des Gehäuses auf der zweiten Seite zu verhindern, wenn eine Kanüle durch das Septum an der ersten Seite des Gehäuses eingeführt wird.

Dieser Durchstechschutz definiert bevorzugt gemeinsam mit einer Wand des Gehäuses einen Kanal. Dazu kann der Durchstechschutz beispielsweise in einem Abstand zur Wand des Gehäuses angeordnet sein, sodass ein flüssigkeitsdurchlässiger Zwischenraum gebildet wird. Ein solcher Zwischenraum definiert einen Kanal, der dazu eingerichtet ist, eine wirkstoffhaltige Flüssigkeit aus dem Speicher zum Auslass zu leiten. Beispielsweise kann ein in einer Flüssigkeit gelöster oder suspendierten Wirkstoff aus dem Speicher durch den Kanal zum Auslass diffundieren.

Der Durchstechschutz kann ein Metall, einen Kunststoff, ein keramisches Material, oder einen Kunststoff-Metall-Verbund aufweisen. In einer Ausführungsform weist der Durchstechschutz ein Material auf, das aufgrund seiner Festigkeit mit einer medizinischen Injektionskanüle gemäß der Norm ISO 7864:2016 nicht durchstochen werden kann.

Der Durchstechschutz kann eine Oberflächenstruktur aufweisen. Diese Oberflächenstruktur kann einen Abstand zwischen dem Durchstechschutz und dem Speicher definieren. Die Oberflächenstruktur kann alternativ oder zusätzlich einen Abstand zwischen dem Durchstechschutz und dem Gehäuse definieren, insbesondere zur zweiten Seite des Gehäuses. Dieser Abstand kann eingerichtet sein, die Diffusion eines gelösten Wirkstoffs um den Durchstechschutz herum zu erlauben, beispielsweise vom Speicher zum Auslass.

Die Oberflächenstruktur kann beispielsweise eine Wölbung eines Teils der Oberfläche des Durchstechschutzes sein. Die Oberflächenstruktur kann bewirken, dass der Durchstechschutz nicht vollständig plan an dem Gehäuse anliegt, sondern an mehreren Stellen freitragende Bereiche aufweist, welche Zwischenräume zwischen dem Durchstechschutz und dem Gehäuse bilden. In ähnlicher Weise kann die Oberflächenstruktur bewirken, dass der Speicher nicht durchgängig plan an der Oberfläche des Durchstechschutzes anliegt.

Der Speicher kann ein offenporige Material aufweisen. Hierdurch kann eine wirkstoffhaltige Flüssigkeit ins Innere des Speichers gelangen, und im Speicher aufgenommen werden.

Der Speicher kann beispielsweise einen retikulierten Schaumstoff, gesinterte Kunststoffpartikel, gepresste Kunststofffasern, gepresste Naturstoffasern, oder verklebte Kunststofffasern aufweisen. Der Schaumstoff kann beispielsweise Polyethylen, Polypropylen, Polyamid, Polyacryl oder Polyurethan umfassen. Geeignete medizinische Schaumstoffe sind u.a. von Porex, Reinbek (Deutschland) erhältlich. Beispiele für Schaumstoffe sind beschrieben in EP2480408A1 und EP1581268A1, welche hiermit durch Bezugnahme vollständig mit aufgenommen werden. Der Schaumstoff kann ein faserhaltiges oder faserfreies Material umfassen oder daraus bestehen.

Der Speicher kann ein Hydrogel aufweisen. Hydrogele enthalten üblicherweise vernetzte Polymere, die eine schwammartige Struktur ausbilden. Der Speicher kann beispielsweise ein vernetztes Salz der Polyacrylsäure, ein vernetztes Polyacrylamid, ein Salz einer vernetzten Polystyrolsulfonsäure, ein Polysaccharid oder ein Polysaccharidderivat aufweisen. Weitere Beispiele umfassen Acrylamid-Copolymere, zum Beispiel ein Copolymer aus Acrylamid und Acrylsäure oder ein Copolymer aus Acrylamid und Natriumacrylat. Weitere Beispiele für Substanzen, die für den Speicher verwendet werden können, sind kommerziell erhältliche Superabsorber wie zum Beispiel HySorb^{®} und SAVIVAO (BASF, Ludwigshafen, Deutschland). Beispiele für Polysaccharide umfassen Cellulose, Guaran, Natriumalginat, Chitosan, Carrageenan, Cyclodextrin und Amylose. Beispiele für Polysaccharidderivate umfassen Carboxymethylcellulose und Carboxymethylamylose.

In einigen Ausführungsformen ist der Speicher innerhalb des Gehäuses angeordnet und eingerichtet, die freie Zirkulation einer wirkstoffhaltigen Flüssigkeit zwischen dem Septum und dem Auslass zu ermöglichen. Dies kann beispielsweise durch eine bewegliche Anordnung des Speichers innerhalb des Gehäuses erzielt werden. Beispielsweise kann der Speicher gegenüber dem Gehäuse, dem Septum und/oder dem Durchstechschutz gegenüber frei beweglich sein. In einigen Ausführungsformen ist der Speicher nicht fest mit dem Gehäuse, dem Septum und/oder dem Durchstechschutz verbunden. Hierdurch kann eine freie Diffusion eines gelösten Wirkstoffs innerhalb des Gehäuses, sowie in, als auch aus dem Speicher, erreicht werden.

In einigen Ausführungsformen weist das Septum ein flexibles, selbstdichtendes Polymer auf. Das Polymer kann ein polyhalogeniertes Olefin, ein Silikon, oder ein thermoplastisches Elastomer aufweisen. Das Septum ist bevorzugt eingerichtet, nach einem Durchstechen des Septums mit einer Kanüle das Gehäuse flüssigkeitsdicht zu verschließen, mit Ausnahme der Auslässe des Gehäuses. Das Septum ist bevorzugt dehnbar und gummielastisch, wie es beispielsweise von Septen in Chemikalienflaschen bekannt ist. Dadurch kann sich das Septum einerseits wie oben beschrieben nach einem Durchstich selbst heilen, und sich bei Injektion einer Flüssigkeit in das Gehäuse ausdehnen, um der indizierten Flüssigkeit ein größeres Volumen innerhalb des Gehäuses bereitzustellen, d. h. durch die Ausdehnung des Septums kann der Hohlraum in dem Gehäuse vergrößert werden. Das Septum kann demnach eingerichtet sein, sich bei Injektion einer Flüssigkeit in den Hohlraum des Gehäuses elastisch zu dehnen.

Bevorzugt weist das Septum ein biokompatibles Material auf.

Das Septum kann beispielsweise eine im Wesentlichen flache oder eine konvex gewölbte Form aufweisen. Das Septum kann eine kreisförmige Form aufweisen.

In einer Ausführungsform weist das Septum ein Material auf, das mit einer medizinischen Injektionskanüle gemäß der Norm ISO 7864:2016 mit einer Kraft von weniger als 100 N durchstechbar ist.

Das Septum ist bevorzugt flüssigkeitsdicht mit dem Gehäuse verbunden.

In einer Ausführungsform weist die zweite Seite des Gehäuses eine im wesentlichen flache Form auf, beispielsweise eine scheibenförmige Form. Auf der Außenseite kann die zweite Seite des Gehäuses eine nach außen gewölbte Strukturen aufweisen, die als Abstandshalter dienen kann. Diese Strukturen können beispielsweise die Form von nach außen hervorstehenden Stegen oder Noppen aufweisen.

Das erfindungsgemäße Implantat kann für eine schnellere oder langsamere Freisetzungskinetik von Wirkstoffen adaptiert werden. Hierzu kann beispielsweise der Innendurchmesser und/oder die Anzahl der Auslässe verringert oder erhöht werden. Demnach kann der Innendurchmesser des Auslasses die Abgabegeschwindigkeit eines Wirkstoffs aus dem Speicher bestimmen. Der Durchmesser eines Auslass kann beispielsweise höchstens 0,5 mm oder höchstens 0,25 mm betragen.

Das hierin beschriebene Implantat kann weiterhin ein Befestigungselement aufweisen, das zur Verbindung mit Körpergewebe eingerichtet ist. Beispielsweise kann an der Außenseite des Gehäuses eine Öse angeordnet sein, die mit einem Zielgewebe vernäht werden kann. Es können auch mehrere solcher Elemente vorgesehen sein, die beispielsweise umlaufend an der Außenseite des Gehäuses angeordnet sein können. Hierdurch kann besser sichergestellt werden, dass eine gezielte lokale Freisetzung eines Wirkstoffs erfolgen kann.

In einigen Ausführungsformen kann der Speicher mit einer wirkstoffhaltigen Flüssigkeit befüllt sein. Beispielsweise kann der Speicher im Rahmen des Herstellungsprozesses bereits mit einer wirkstoffhaltigen Flüssigkeit vorbefüllt werden. Dies kann besser gewährleisten, dass die wirkstoffhaltige Flüssigkeit bis zur Anwendung steril bleibt, und eine exakt vorbestimmte Menge eines Wirkstoffs im Implantat enthalten ist. Zudem kann sich auf diese Weise besser ein Gleichgewicht der Konzentration des Wirkstoffs zwischen dem Speicher und dem sonstigen Hohlraum innerhalb des Gehäuses einstellen. Die Befüllung des Speichers kann wie hierin beschrieben durch Injektion durch das Septum geschehen. Alternativ kann ein vorbefüllter Speicher in das Gehäuse eingebaut werden, sodass keine Verletzung des Septums während des Herstellungsprozesses erfolgen muss.

Speicher kann grundsätzlich mit jedem Wirkstoff gefüllt werden, der in einer Flüssigkeit gelöst oder suspendiert werden kann. Besonders bevorzugt sind antirheumatische Wirkstoffe. Beispielsweise stehen für die Therapie der rheumatoiden Arthritis und der Psoriasisarthritis eine Reihe von Wirkstoffgruppen zur Verfügung. Diese Wirkstoffgruppen umfassen Analgetika, nichtsteroidale Antiphlogistika, Glucocorticoide und Basistherapeutika (Disease modifying antirheumatic drugs, DMARD). Daneben gibt es therapeutische Antikörper (Biologicals), welche die Signalwege von α-TNF (α-Tumornekrosefaktor) oder Interleukin IL-17 inhibieren. Weiterhin verwendbar sind niedermolekulare Wirkstoffe, die das Immunsystem supprimieren. Solche Immunmodulatoren umfassen beispielsweise Cyclosporin A (CAS 59865-13-3), Tacrolimus (CAS 104987-11-3) und Everolimus (CAS 159351-69-6). Weitere Beispiele für Wirkstoffe sind Cortison (CAS 53-06-5), Betamethason (CAS 378-44-9), Triamcinolon (CAS 124-94-7), Dexamethason (CAS 50-02-2),Dexamethasonphosphat (CAS 2392-39-4) und Apremilast (CAS 608141-41-9).

Der Wirkungsmechanismus von Cyclosporin A ist auf die Bildung eines Komplexes aus Cyclosporin A und dem Immunophilin Cyclophilin zurückzuführen. Dieser Komplex inhibiert die Phosphatase Calcineurin. Das Enzym Calcineurin ist wiederum essentiell für die Bildung von α-TNF, wobei α-TNF eine Schlüsselverbindung am Beginn der Entzündungskaskaden ist. Durch Inhibieren der Synthese von α-TNF können demzufolge die nachfolgenden Entzündungskaskaden ebenfalls inhibiert werden.

Bei der systemischen medikamentösen Therapie können Serum-Wirkstoffkonzentrationen für Cyclosporin A im Bereich von ca. 75 µg/l bis 175 µg/l und für Tacrolimus von 5 µg/l bis 20 µg/l erreicht werden. Höhere systemische Wirkstoffkonzentrationen sind auf Grund der Gefahr von unerwünschten toxischen Nebenwirkungen für den restlichen Organismus systemisch für eine länger andauernde Therapie nicht möglich. Das bedeutet, dass im Gewebe der Gelenkkapsel (Membrana fibrosa und Membrana synovialis) die Wirkstoffkonzentrationen bei systemischer Therapie sehr niedrig sind und daher nur einen begrenzten immunmodulierenden Effekt auf das Entzündungsgeschehen haben können. Mithilfe des erfindungsgemäßen Implantats dagegen können zur Verminderung der chronischen Entzündungsprozesse im Gelenkbereich von Patienten mit rheumatoider Arthritis oder Psoriasisarthritis Wirkstoffe direkt am Gewebe der Gelenkkapsel oder in der Nähe der Gelenkkapsel subkutan appliziert werden, um eine lokal ausreichend hohe Konzentration dieser Wirkstoffe zu erreichen.

Das Implantat kann auch nach der Implantation im Patienten mit Wirkstoff befüllt werden. Wenn das Implantat beispielsweise subkutan implantiert ist, kann der Speicher durch Injektion durch das Septum von außen befüllt werden, ohne dass das Implantat freigelegt oder explantiert werden muss. Eine weitere Ausführungsform beschreibt demgemäß ein Implantat, welches eingerichtet ist, in einem subkutan implantierten Zustand mit einer wirkstoffhaltigen Flüssigkeit befüllt zu werden, indem die Flüssigkeit durch das Septum in das Gehäuse injiziert wird. Die Flüssigkeit kann dabei in den Hohlraum des Gehäuses injiziert werden, oder bevorzugt direkt in den Speicher, der in dem Hohlraum des Gehäuses angeordnet ist.

Ein weiterer Aspekt der Erfindung betrifft ein Verfahren zum Befüllen eines Implantats, wobei das Implantat einen Speicher und ein Septum umfasst, wobei das Septum in einem implantierten Zustand des Implantats von außen mit einer Kanüle durchstochen wird, um eine wirkstoffhaltige Flüssigkeit in den Speicher einzubringen. Die Flüssigkeit kann hierbei direkt in den Speicher injiziert werden, oder kann außerhalb des Speichers in den Hohlraum eingebracht werden, und danach durch den Speicher aufgenommen werden. Nach dem Injektionsvorgang kann das Septum sich durch die Rückstellkraft des elastischen Materials des Septums wieder selbst flüssigkeitsdicht verschließen. In einer Ausführungsform wird das Implantat in einem nicht implantierten Zustand befüllt, d. h. *in vitro.*

Ein weiterer Aspekt betrifft ein medizinisches Behandlungsverfahren, wobei mithilfe eines hierin beschriebenen Implantats ein Wirkstoff verzögert an ein Gelenk abgegeben wird. Das Implantat kann hierzu beispielsweise subkutan im Bereich eines Gelenks implantiert und gegebenenfalls mit Weichgewebe verbunden werden, beispielsweise mithilfe der hierin beschriebenen Befestigungselemente.

Ein weiterer Aspekt der Erfindung betrifft einen antirheumatischen Wirkstoff zur Anwendung in einem Therapieverfahren. Der Wirkstoff kann hierbei mithilfe der hierin beschriebenen Implantate und Verfahren verabreicht werden. Der Wirkstoff kann dabei verzögert aus einem Speicher über einen Auslass des Implantats abgegeben werden, wie hierin beschrieben.

### BEISPIELE

Die Erfindung wird nachfolgend anhand von Beispielen weiter verdeutlicht, die jedoch nicht als einschränkend zu verstehen sind. Dem Fachmann wird ersichtlich sein, dass anstelle der hier beschriebenen Merkmale andere äquivalente Mittel in ähnlicher Weise verwendet werden können.

### FIGUREN

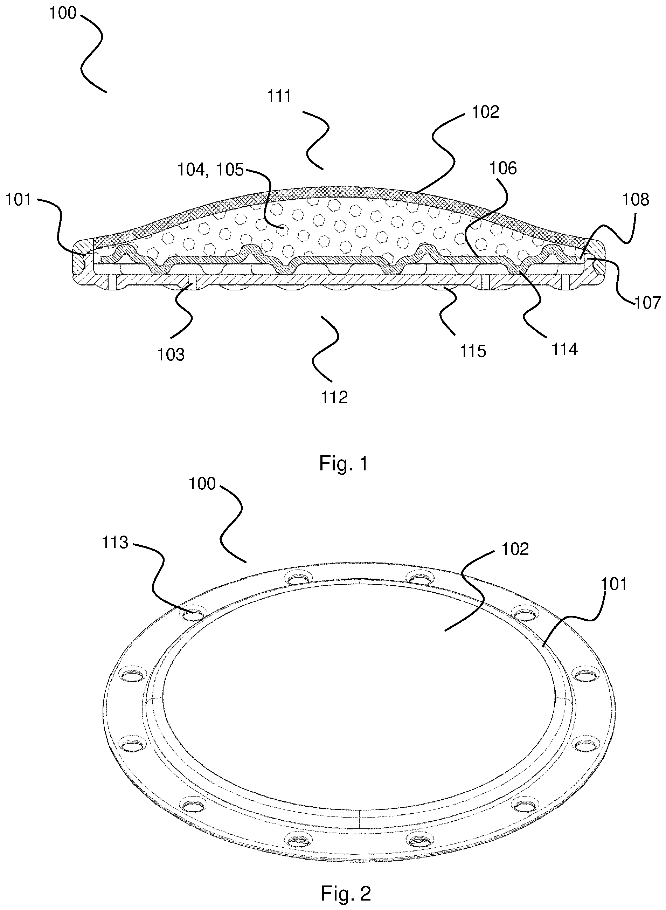

**Fig. 1** zeigt beispielhaft eine erste Ausführungsform eines erfindungsgemäßen Implantats **100** in einer Querschnittsansicht. Das Implantat weist ein scheibenförmige Gehäuse **101** auf, welches in dem hier gezeigten Beispiel zweiteilig ausgebildet ist. An einer ersten Seite **111** ist in den oberen Teil des Gehäuses ein Septum **102** flüssigkeitsdicht eingebracht. In den unteren Teil des Gehäuses **101** sind an einer zweiten Seite des Gehäuses **112** Auslässe **103** eingebracht, welche den Hohlraum **105** im Inneren des Gehäuses flüssigkeitsleitend mit der äußeren Umgebung des Gehäuses verbinden. In dem Hohlraum **105** des Gehäuses **101** ist weiterhin ein Speicher **104** angeordnet, der zur Aufnahme einer wirkstoffhaltigen Flüssigkeit eingerichtet ist. Zwischen dem Speicher **104** und der zweiten Seite des Gehäuses **112** ist ein Durchstechschutz **106** angeordnet, welcher das Durchstechen mit einer Injektionskanüle verhindern kann. Der Speicher weist ein Material mit offener Porosität auf, die ein Eindringen und Speichern von Flüssigkeit erlaubt. Der Durchstechschutz kann beispielsweise aus einem festen Metall, wie zum Beispiel medizinischem rostfreiem Stahl oder Titan, gebildet sein. Der Durchstechschutz **106** ist zu einer seitlichen Wand **107** des Gehäuses beabstandet, um dazwischen einen Kanal **108** auszubilden. Der Kanal **108** erlaubt den Transport von Wirkstoffen von der ersten Seite **111** auf die zweite Seite **112** des Hohlraums **105,** sodass Wirkstoffe durch die Auslässe **103** nach außen abgegeben werden können.
**Fig. 2** zeigt eine weitere Ausführungsform eines erfindungsgemäßen Implantats **100** in einer isometrischen Aufsicht. Das Implantat weist auf der Oberseite ein Septum **102** auf. Das Gehäuse **101** weist umlaufend um den seitlichen Rand mehrere Befestigungselemente **113** auf, die hier als Löcher in einem seitlich hervorstehenden Teil des Gehäuses **101** ausgebildet sind. Die Befestigungselemente **113** erlauben eine Fixierung des Implantats an einem Zielgewebe, zum Beispiel das Vernähen des Implantats mit Weichgewebe im Bereich eines Gelenks.

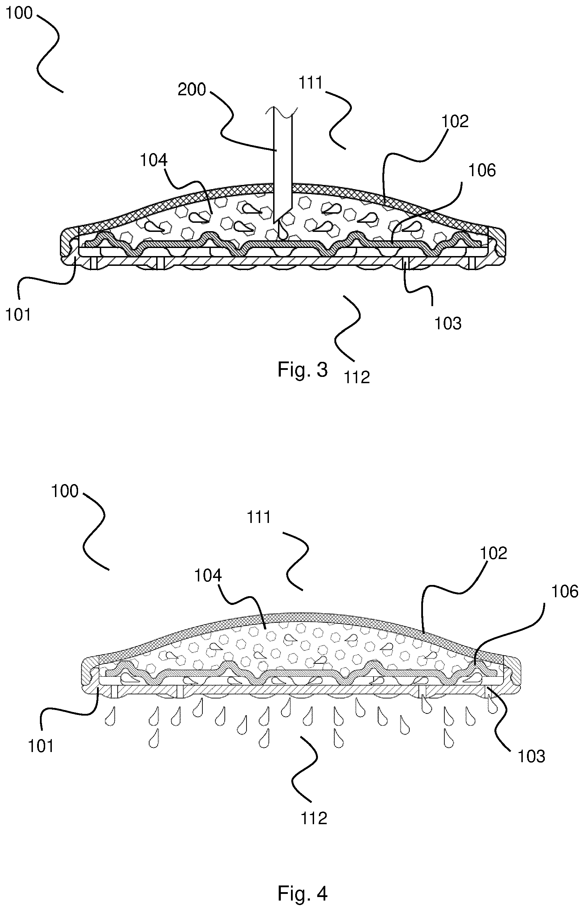

**Fig. 3** zeigt die Befüllung eines erfindungsgemäßen Implantats **100** mithilfe einer Kanüle **200** in einer Querschnittansicht. Hierbei wird mithilfe einer Kanüle **200** das Septum **102** durchstochen, und eine wirkstoffhaltige Flüssigkeit (in der Abbildung sinnbildlich als Tropfen dargestellt) in das Gehäuse eingebracht. Die Flüssigkeit kann gleichzeitig oder nachfolgend in den Speicher **104** aufgenommen werden. Der Durchstechschutz **106** verhindert, dass die zweite Seite **112** des Gehäuses **101** von der Kanüle **200** durchstochen wird. Hierdurch wird das darunterliegende Gewebe geschützt, wenn die Befüllung des Implantats im implantierten Zustand vorgenommen wird.
**Fig. 4** zeigt die Abgabe einer wirkstoffhaltigen Flüssigkeit aus einem erfindungsgemäßen Implantat **100** in einer Querschnittansicht. Die Flüssigkeit und/oder ein darin gelöster Wirkstoff wird aus dem Speicher **104** an den Hohlraum des Gehäuses **101** abgegeben, und gelangt durch die Auslässe **103** auf der zweiten Seite **112** des Gehäuses **101** nach außen. Der Durchstechschutz **106** ist so angeordnet, dass er den Transport des Wirkstoffs vom Speicher **104** zu den Auslässen **103** erlaubt.

### BEZUGSZEICHENLISTE

- 100: Implantat
- 101: Gehäuse
- 102: Septum
- 103: Auslass
- 104: Speicher
- 105: Hohlraum
- 106: Durchstechschutz
- 107: Wand
- 108: Kanal
- 111: erste Seite des Gehäuses
- 112: zweite Seite des Gehäuses
- 113: Befestigungselement
- 114: Oberflächenstruktur
- 200: Kanüle

## Patentansprüche

1. Implantat (100) zur subkutanen verzögerten Wirkstofffreisetzung, aufweisend ein Gehäuse (101), welches an einer ersten Seite (111) durch ein Septum (102) begrenzt ist, und an einer zweiten Seite (112) einen Auslass (103) zur Wirkstofffreisetzung aufweist, und einen Speicher (104), der in einem Hohlraum (105) des Gehäuses (101) angeordnet und zur Aufnahme einer wirkstoffhaltigen Flüssigkeit eingerichtet ist.

2. Implantat nach Anspruch 1, wobei das Implantat innerhalb des Gehäuses (101) weiterhin einen Durchstechschutz (106) aufweist, der gemeinsam mit einer Wand (107) des Gehäuses einen Kanal (108) definiert, wobei der Kanal (108) eingerichtet ist, Wirkstoff aus dem Speicher (104) zum Auslass (103) zu leiten.

3. Implantat nach Anspruch 2, wobei der Durchstechschutz (106) eine Oberflächenstruktur (114) umfasst, welche einen Abstand des Durchstechschutz (106) zu dem Speicher (104) und/oder zu dem Gehäuse (101) definiert.

4. Implantat nach einem der vorangehenden Ansprüche, wobei der Speicher (104) ein offenporiges Material aufweist.

5. Implantat nach einem der vorangehenden Ansprüche, wobei der Speicher (104) einen retikulierten Schaumstoff, gesinterte Kunststoffpartikel, gepresste Kunststofffasern, gepresste Naturstoffasern, oder verklebte Kunststofffasern aufweist.

6. Implantat nach einem der vorangehenden Ansprüche, wobei der Speicher (104) ein vernetztes Salz der Polyacrylsäure, ein vernetztes Polyacrylamid, ein Salz einer vernetzten Polystyrolsulfonsäure, ein Polysaccharid oder ein Polysaccharidderivat aufweist.

7. Implantat nach einem der vorangehenden Ansprüche, wobei der Speicher (104) innerhalb des Gehäuses (101) angeordnet und eingerichtet ist, die freie Zirkulation einer wirkstoffhaltigen Flüssigkeit zwischen dem Septum (102) und dem Auslass (103) zu ermöglichen.

8. Implantat nach einem der vorangehenden Ansprüche, wobei das Septum (102) ein flexibles, selbstdichtendes Polymer aufweist, wobei das Polymer bevorzugt ein polyhalogeniertes Olefin, ein Silikon, oder ein thermoplastisches Elastomer aufweist.

9. Implantat nach einem der vorangehenden Ansprüche, wobei das Septum (102) eine konvex gewölbte Form aufweist.

10. Implantat nach einem der vorangehenden Ansprüche, wobei das Septum (102) eingerichtet ist, sich bei Injektion einer Flüssigkeit in den Hohlraum (105) des Gehäuses (101) elastisch zu dehnen.

11. Implantat nach einem der vorangehenden Ansprüche, wobei die zweite Seite (112) des Gehäuses (101) eine im wesentlichen flache Form aufweist.

12. Implantat nach einem der vorangehenden Ansprüche, wobei der Innendurchmesser des Auslasses (103) die Abgabegeschwindigkeit eines Wirkstoffs aus dem Speicher (104) bestimmt.

13. Implantat nach einem der vorangehenden Ansprüche, welches weiterhin ein Befestigungselement (113) aufweist, das zur Verbindung mit Körpergewebe eingerichtet ist.

14. Implantat nach einem der vorangehenden Ansprüche, wobei der Speicher (104) mit einer wirkstoffhaltigen Flüssigkeit befüllt ist.

15. Implantat nach einem der vorangehenden Ansprüche, welches eingerichtet ist, in einem subkutan implantierten Zustand mit einer wirkstoffhaltigen Flüssigkeit befüllt zu werden, indem die Flüssigkeit durch das Septum (102) in das Gehäuse (101), bevorzugt in den Speicher (104), injiziert wird.
